Europäisches Patentamt
European Patent Office
Office européen des brevets

(11) Publication number: **0 363 326 A2**

# EUROPEAN PATENT APPLICATION

(21) Application number: 89810756.0

(51) Int. Cl.5: **A01N 33/04 , C07C 211/30**

(22) Date of filing: 04.10.89

(30) Priority: 07.10.88 GB 8823614
26.05.89 GB 8912235

(43) Date of publication of application:
11.04.90 Bulletin 90/15

(84) Designated Contracting States:
AT BE CH DE ES FR GB GR IT LI LU NL SE

(71) Applicant: SANDOZ AG
Lichtstrasse 35
CH-4002 Basel(CH)
(84) BE CH ES FR GB GR IT LI LU NL SE

Applicant: SANDOZ-PATENT-GMBH
Humboldtstrasse 3
D-7850 Lörrach(DE)
(84) DE

Applicant: SANDOZ-ERFINDUNGEN
Verwaltungsgesellschaft m.b.H.
Brunner Strasse 59
A-1235 Wien(AT)
(84) AT

(72) Inventor: Stähle-Csech, Ursula
Riehenerstrasse 30/2
D-7858 Weil(DE)
Inventor: Stütz, Anton
Lindauergasse 35
A-1238 Vienna(AT)

(54) **Fungicidal amines.**

(57) The present invention relates to the use of naphthylmethyl amines of formula I

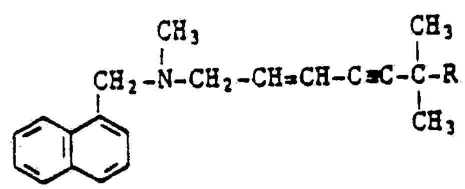

I

wherein R is $CH_3$, $C_2H_5$ or n-$C_3H_7$,
in free base form or in agriculturally acceptable acid addition salt form, suitable for use as seed dressing agents and soil application.

EP 0 363 326 A2

## FUNGICIDAL AMINES

The present invention relates to the use of naphthylmethyl amines against soil- and seed-borne diseases.

It has been found that compounds of formula I

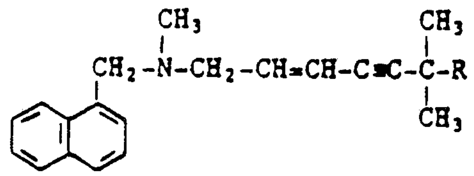

I

wherein R is $CH_3$, $C_2H_5$ or n-$C_3H_7$,
in free base form or in agriculturally acceptable acid addition salt form are suitable for use as seed dressing agents and soil application.

The compounds of formula I in which R is $CH_3$ and $C_2H_5$ are disclosed as examples number 16 and 49 resp. in EPA 0024587. Said application discloses generically but not specifically, the compound of formula I in which R is n-$C_3H_7$. If also discloses processes for the production of the compounds of formula I, pharmaceutical compositions containing them and their use as pharmaceuticals. The compounds of formula I in which R is $CH_3$ is known as a pharmaceutical under the common name terbinafine.

P. Leroux et al disclose in C.R. Acad. Sc. Paris, t. 301, Série III, (17) 785-788 (1985) the fungicidal activity of naftifine and terbinafine in in-vitro tests against Pseudocercosporella herpotrichoides, the causal agent of eyespot of cereals.

Spray treatment of plants with compounds belonging to the general class of the compounds of formula I do not however provide the desired fungal control, and in general the level of control in field tests is substantially lower than that one would expect on the basis of greenhouse test results. Furthermore, the level of fungicidal activity observed in greenhouse tests with compounds of formula I against air-borne fungi is not sufficient to warrant commercial interest.

It has now been found that the compounds of formula I, in free form or in agriculturally acceptable acid addition salt form are surprisingly effective as control agents against seed and soil-borne diseases when used in solid application form, in particular when used as active ingredient in a seed dressing or in a granular formulation form.

The solid application forms comprising compounds of formula I, in free form or in agriculturally acceptable acid addition salt form, are particularly suitable for use against the seed-borne diseases caused by fungi, more particularly against Ustilago spp, Tilletia spp, Fusarium spp (including Gerlachia spp), Pyrenophora spp (including Helminthosporium, Cochliobolus, Drechslera) and against Septoria spp (the latter diseases may also be soil-borne), and against the soil-borne fungus Pseudocercosporella herpotrichoides, as indicated by tests.

The efficacy against seed-borne diseases can be illustrated by the inhibition zone test: single seeds treated with a seed dressing formulation comprising the compound to be tested e.g. with the seed dressing formulation according to Example 2 (hereinafter described) are placed on agar containing the seed-borne fungus. The test substance, which diffuses from the treated seed into the agar medium, will induce an inhibition zone around the seed the size of which is in direct relation with the fungicidal activity of the test substance. The size of the inhibition zone is evaluated one week after the seed has been placed on the agar. The activity is expressed as the concentration (in g per 100 kg seed) resulting in 90 % or more growth inhibition of the fungal mycelium.

The test results are summarized in the following tables;

Table I

| Barley seeds treated with a seed dressing comprising a compound of formula I | | | |
|---|---|---|---|
| | Concentration (g/100 kg Seed) providing ≧90% control | | |
| Disease / Test subst. R | Pyrenophora teres | Pyrenophora graminea | Cochliobulus sativus ** |
| $CH_3$ (*) | nt | <5 | nt |
| $C_2H_5$ | 4 | 4 | 4 |
| $nC_3H_5$ | 4 | 2 | 2 |
| Standard A (Imazalil) | 8 | 8 | 4 |

* in hydrochloride acid addition salt form.
** also known under the names Helminthosporium sativum, Helminthosporium teres, Pyrenophora teres and Drechslera teres.
nt = not tested

Table II

| Wheat seeds treated with a seed dressing comprising a compound of formula I | | | |
|---|---|---|---|
| | Concentration (g/100 kg Seed) providing ≧90% control | | |
| Disease / Test subst. R | Gerlachia nivalis | Fusarium culmorum | Septoria nodorum |
| $CH_3$ (*) | <5 | nt | nt |
| $C_2H_5$ | 4 | >8 | 2 |
| $nC_3H_7$ | 2 | 8 | 2 |
| Standards | | | |
| (Imazalil) | nt | nt | 8 |
| (Guazatine) | 8 | 4 | nt |

* in hydrochloride acid addition salt form.
nt = not tested

The seed dressing formulation is applied in a manner know per se to the seeds employing the compound of formula I and a diluent in suitable seed dressing formulation form, e.g. as an aqueous suspension or in a dry powder form having good adherance to the seeds. Such seed dressing formulations are known in the art. Typical examples (in which parts are by weight) are shown below.

Seed Dressing Formulation Example 1

25 parts of a compound of formula I, in free form or acid addition salt form,
15 parts of dialkylphenoxypoly(ethylenoxy)ethanol

15 parts of fine silica
44 parts of fine kaolin
0.5 parts of Rhodamin B as a colorant and
0.5 parts of Xanthan Gum
are mixed and ground in a contraplex mill at approx. 10'000 rpm to an average particle size of below 20 microns. The resulting formation is applied to the seeds as an aqueous suspension in an apparatus suitable for that purpose.

Where the compound of formula I is liquid, it is first absorbed on the carriers, if desired with the aid of a small amount of a volatile solvent such as dichloromethane. The resulting power is first allowed to dry if a solvent was used, then the other ingredients are added and the rest of the procedure is carried out.

Seed Dressing Formulation Example 2

45 Parts of a compound of formula I, in free form or acid addition salt form are mixed with 1.5 parts of diamyl phenoldecaglycolether ethylene oxide adduct, 2 parts of spindle oil, 51 parts of fine talcum and 0.5 parts of colorant Rhodamin B. The mixture is ground in a contraplex mill at 10'000 rpm until an average particle size of less than 20 microns is obtained. The resulting dry powder has good adherance and may be applied to seeds, e.g. by mixing for 2 to 5 minutes in a slowly turning vessel.

The above test results indicate a broad spectrum of activity against seed-borne fungal diseases, in particular against such diseases in cereals, including i.a. wheat and barley. The useful level of activity is confirmed by indoor and outdoor pot tests.

Indoor Pot Test

Spring barley seed naturally infested by Pyrenophora graminea are treated with a seed dressing formulation according to Example 1 and sown in pots filled with a substrate equivalent to a sandy loam in a growth chamber at 10 °C with 14-17 hours daylight. The determination of the fungal control involves a visual evaluation 60 days after said dressing and sowing. Fungicidal activity is observed (Table III).

Outdoor Pot Test

Analogous tests are run outdoors under naturally occurring wheather conditions with spring barley infected by Pyrenophora graminea and Ustilago nuda. Fungicidal activity is observed (Table III).

Table III

| Pot Tests | | | | |
|---|---|---|---|---|
| | | % Control | | |
| Compound of formula I R | dosage g a.i./100kg seed | indoors | outdoors | |
| | | P. gram. | P. gram. | U. nuda |
| $C_2H_5$ | 15 | 75-80 | 60-95 | 100 |
| $nC_3H_7$ | 8 | 80-90 | 80 | 100 |

In view of the test results, the compounds of formula I and their agriculturally acceptable acid addition salts are indicated for use as seed dressing agents and soil application. In general, satisfactory control of seed-borne diseases is obtained when employing from 10 to 30 g of a compound of formula I, or an acid addition salt thereof, per 100 kg of seed. It has furthermore been observed that seedlings of thus treated seeds show a reduced susceptibility to soil-borne diseases, in particular against Pseudocercosporella herpotrichoides. Thus cereals of which the seeds are coated with a seed dressing formulation comprising a compound of formula I in free form or agriculturally acceptable acid addition salt form may not require the

first post emergence treatments usually necessary for preventing or combatting cereal soil-borne diseases. Such favourable activity is in particular observed against cereal foot diseases, more particularly against Pseudocercosporella herpotrichoides.

The compounds of formula I, in free base form or agriculturally acceptable acid addition salt form are surprisingly effective against soil-borne diseases in particular soil-borne diseases of cereals, more particularly against cereal foot diseases, more particularly against Pseudocercosporella herpotrichoides when applied to the soil of the crop locus in solid formulation form. In general satisfactory results are observed with application rates of from 100 to 1000 g active ingredient, preferably of from 200 to 600 g active ingredient per hectare of crop locus.

Solid formulation forms suitable for use in the method of the invention against soil-borne diseases comprise any conventional solid formulation suitable for application in undiluted form such as dusts and granules. They are obtained in conventional manner e.g. by admixing a compound of formula I in free base form or in agriculturally acceptable acid addition salt form with an agriculturally acceptable solid carrier and, optionally, additional excipients such as a binding agent. Examples of appropriate carriers comprise talc, kaolin, quartz sand and diatomaceous earth.

The solid formulations of the invention comprise conveniently from 0.5 % to 20 % by weight of a compound of formula I, in free base form or agriculturally acceptable acid addition salt form and 80 % to 99.5 % agriculturally acceptable carrier and adjuvants.

Further active ingredients, e.g. fungicides with similar or complementary fungicidal activity or other beneficially-acting materials, such as insecticides may be present in the formulation.

An example of a suitable solid formulation for use against soil-borne fungi is as follows:

Granules

0.5 Parts by weight of a binder (a non-ionic tenside) are sprayed onto 94.5 parts by weight of quartz sand in a tumbler mixer and thoroughly mixed. 5 Parts by weight of a compound of formula I are then added and thorough mixing continued to obtain a granulate formulation with a particle size in the range of from 0.3 to 0.7 mm.

This invention further provides a process for preparing the compound of formula I, in which R is n-$C_3H_7$ (hereinafter Compound A), in free base form or agriculturally acceptable acid addition salt form. Compound A may be obained in a manner known per se for N-(2-alken-4-inyl)-N-Methyl-1-naphthylmethylamines.

An appropriate method of preparing Compound A comprises for example N-alkylating N-methyl-1-naphthylmethylamine with a compound of formula II

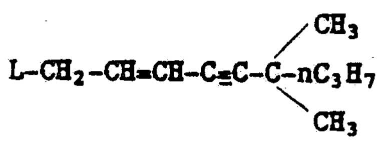

II

wherein L is a leaving group.

The process for the production of Compound A may be effected in conventional manner for the production of tertiary amines by condensation from secondary amines. The process may be effected in an inert solvent such as a lower alkanol, e.g. ethanol, optionally in aqueous admixture, an aromatic hydrocarbon solvent, e.g. benzene or toluene, a cyclic ether, e.g. dioxane or a carboxylic acid dialkylamide solvent, e.g. dimethylformamide. The reaction temperature is conveniently from room temperature to the boiling temperature of the reaction mixture, preferably room temperature. The reaction is conveniently effected in the presence of an acid binding agent, such as an alkali metal carbonate, e.g.. sodium carbonate. The leaving group L is conveniently iodine or preferably chlorine or bromine, or an organic sulphonyloxy group having 1 to 10 carbon atoms, e.g. alkylsulphonyloxy, preferably having 1 to 4 carbon atoms such as mesyloxy, or alkylphenylsulphonyloxy preferably having 7 to 10 carbon atoms such as tosyloxy.

Free base forms of Compound A may be converted into salt forms and vice versa. Suitable acid addition salts are e.g. hydrochloride, hydrogen fumarate or naphthaline-1,5-disulphonate.

The compounds of the formula I and their intermediates can be obtained in the form of isomeric mixtures of the various cis/trans isomers which can be separated according to established methods. Alternatively, isomers of the compounds can be obtained by using the appropriate isomer of the starting material. Unless otherwise stated the compounds are always to be understood as being mixtures of these

isomers.

**EXAMPLE 1 - N-(6,6-Dimethyl-2-nonen-4-inyl)-N-Methyl-1-naphthylmethylamine (CompoundA)**

To a mixture of 1.28 g N-methyl-1-naphthylmethylamine, 1.2 g $K_2CO_3$ and 50 ml dimethylformamide are added dropwise 1.7 g 1-bromo-6-dimethyl-2-nonen-4-ine (E/Z = 3/1). The mixture is stirred over night, the solvent then distilled off in vacuo and the residue distributed between ether and an aqueous $NaHCO_3$ solution. The organic phase is dried, evaporated and chromatographed over silica gel (hexanol/ethylacetate 9:1) whereby first the (E)-isomer and thereafter the (Z)-isomer is eluated. Both isomers are oils:
**NMR ($CDCl_3$):**
**(E):** 8,2-8,4 (m, 1H); 7,7-7,96 (m, 2H); 7.36-7,64 (m,4H); 6,16 (dt, J=16+6 Hz,1H); 5,68 (dt, J= 16+1,5 Hz, 1H); 3,9 (s,2H); 3,14 (dd, J=6+1,6 Hz, 2H); 2,22 (s,3H); 1,3-1,52 (m,4H); 1,19 (s, 6H); 0.92 (tr, J=7 Hz,3H).
**(Z):** 8,2-8,42 (m,1H); 7,36-8,0 (m,6H); 6,06 (dt, J=12+6 Hz, 1H); 5,67 (dt, J=12+1,5 Hz, 1H); 3,94 (s,2H); 3,38 (dd, J=6+1,5 Hz, 2H); 2,26 (s,3H); 1,35-1,56 (m,4H); 1,23 (s,6H); 0,94 (tr, J=7 Hz,3H).

**Preparation of the intermediates:**

**a) 6,6-Dimethyl-1-nonen-4-in-3-ol**

To a solution of 2,95 g 3,3-dimethyl-1-hexine in 40 ml abs. tetrahydrofuran are added dropwise, under $N_2$, at -20°C, 16.7 ml of a 20 % solution of n-butyllithium. The mixture is cooled to -75°C and 1.5 g acroleine in 20 ml abs. tetrahydrofuran are then added dropwise. The reaction mixture is heated to room temperature, a saturated aqueous $NH_4Cl$ solution is added and the mixture extracted several times with diethylether.

The organic phase is dried and evaporated. The thus obtained title compound is reacted further without prior purification.

**b) (E) and (Z) 1-Bromo-6,6-dimethyl-2-nonen-4-ine**

15 ml 48 % HBr and 3 g $PBr_3$ are stirred at 40°C till the mixture is homogeneous. Thereto is added, at 10°C, an alcoholic solution of 2.45 g 6,6-dimethyl-1-nonen-4-in-3-ol and the mixture is stirred for 1.5 hours. The reaction mixture is poured onto ice and extracted several times with hexane. The organic phase is washed repeatedly with aqueous NaCl solution, dried and evaporated. The NMR-spectrum indicates a (E)-/(Z) mixture of the title compound of 3/1, which mixture is used as such in the process of Example 1.
**NMR ($CDCl_3$):**
5,4-6,4 (mm, 2 olef.H); [4,15 (d, J=7,5 Hz) and 3,93 (d, J=7,5 Hz) in the ratio 1:3, 2H, =CH-$CH_2$Br]; 1,3-1,55 (m,4H); [1,25 (s) and 1,2 (s) in the ratio 1:3, 6H, -$C(CH_3)_2$-]; 0.9 (tr, J=7 Hz, 3H).

**Claims**

1. Method of combatting seed or soil borne fungal diseases which comprises applying to the seeds or soil to be treated a fungicidally effective amount of a compound of formula I

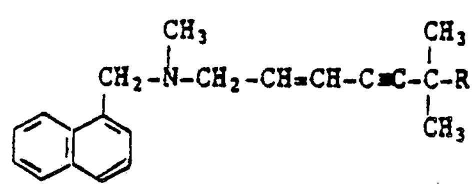

(I)

wherein R is $CH_3$, or $C_2H_5$ or n-$C_3H_7$ in free base form or in agriculturally acceptable acid addition salt form.

2. A seed dressing agent of a compound of formula I, stated in Claim 1, in free form or in agriculturally

acceptable acid addition salt form.

3. Seeds coated with a seed dressing formulation comprising a fungicidally effective amount of a compound of formula I, stated in Claim 1, in free form or in agriculturally acceptable acid addition salt form.

4. A solid fungicidal formulation for combatting soil borne diseases by treatment of the soil, comprising a fungicidally effective amount of a compound of formula I, stated in Claim 1, in free form or in agriculturally acceptable acid addition salt form.

5. The compound of formula I, stated in Claim 1, wherein R is n-$C_3H_7$, in free base form or acid addition salt form.

6. Process of preparing the compound of formula I, stated in Claim 1, wherein R is n-$C_3H_7$, in free base form or acid addition salt form which comprises N-alkylating N-methyl-1-naphthylmethylamine with a compound of formula II

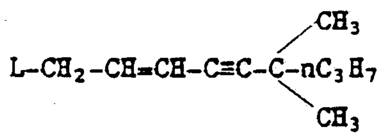

II

wherein L is a leaving group,
and optionally converting the thus obtained free base into acid addition salt forms thereof.